# EUROPEAN PATENT APPLICATION

(11) **EP 2 767 219 A1**
(43) Date of publication of application: **20.08.2014**
(21) Application number: 14155202.6
(22) Date of filing: 14.02.2014
(51) Int. Cl.: A61B 3/032, G02B 27/02, G09B 25/00

(54) **A system for determining a recommended magnification factor for a magnifier such as a loupe or an electronic magnifier to be used by a person**

(30) Priority: 14.02.2013 NL 2010302
(71) Applicant: Optelec Development B.V., 2993 LT Barendrecht (NL)
(72) Inventor: Auger, Robert, 2993 TL Barendrecht (NL)
(74) Representative: Jansen, Cornelis Marinus

(57) **Abstract**

A system for determining a recommended magnification factor and/or a recommended range for magnification factors for a magnifier such as a loupe or an electronic magnifier to be used by a person wherein the system comprises at least one test object having a predetermined size and a portable camera for recording an image of at least a portion of the at least one test object and displaying at least a portion of the image of the at least one test object to the person, wherein the camera is provided with a light sensitive sensor for obtaining an image of at least a portion of the at least one test object, a display and processing means for processing the image obtained by means of the sensor and for displaying at least a portion of the image of the at least one test object on the display, in possible a magnified form, wherein the camera is provided with means for selecting the magnification of the at least one portion of the image which is displayed on the display and wherein the camera is arranged to calculate the recommended magnification factor and/or the recommended range for magnification factors on basis of a predetermined algorithm wherein the predetermined algorithm uses as parameters: a value representing the selected magnification for the displayed at least one portion of the image, the size of the at least one test object and a distance between an eye of the person and the at least one test object while the at least one test object is seen by the person by means of the portable camera; and/or parameters which can be derived from the value representing the selected magnification for the displayed at least one portion of the image, the size of the at least one test object and a distance between an eye of the person and the object while the at least one test object is seen by the person by means of the portable camera.

## Description

The present invention relates to a system for determining a recommended magnification factor and/or the recommended range for magnification factors for a magnifier such as a loupe or an electronic magnifier to be used by a person. Such systems are generally known and is also referred to as vision assessment. In the known tests, certain charts such as tumbling E-chart and the Landolt C chart are commonly used. Such charts are provided with a plurality of symbols having different sizes. In such a test a person is positioned at a predetermined distance from the test chart. Then it is checked which symbols can be clearly viewed and/or recognized by the person using one or both eyes. The smallest symbol which can still be clearly viewed and/or recognized by the person represents a quality of the eye or the eyes. Thus, it is determined if the smallest size of a character can still be clearly viewed and/or recognized. If such symbol appears to be relatively large, then such a person may wish to use a magnifier such as a loupe or an electronic magnifier for viewing such characters on printed paper. Depending on the size of the smallest symbol which can still be clearly viewed and/or recognized by the person in the test, a recommended magnification factor for a magnifier such as a loupe or an electronic magnifier can be determined.

The present invention provides a system for carrying out such a test in an improved manner.

The system according to the invention is characterized in that the system comprises at least one test object having a predetermined size and a portable camera for recording an image of at least a portion of the at least one test object and displaying at least a portion of the image of the at least one test object to the person, wherein the camera is provided with a light sensitive sensor for obtaining an image of at least a portion of the at least one test object, a display and processing means for processing the image obtained by means of the sensor and for displaying at least a portion of the image of the at least one test object on the display, in possible a magnified form, wherein the camera is provided with means for selecting the magnification of the at least one portion of the image which is displayed on the display and wherein the camera is arranged to calculate the recommended magnification factor and/or the recommended range for magnification factors on basis of a predetermined algorithm wherein the predetermined algorithm uses as parameters: a value representing the selected magnification for the displayed at least one portion of the image, the size of the at least one test object and a distance between an eye of the person and the at least one test object while the at least one test object is seen by the person by means of the portable camera; and/or parameters which can be derived from the value representing the selected magnification for the displayed at least one portion of the image, the size of the at least one test object and a distance between an eye of the person and the object while the at least one test object is seen by the person by means of the portable camera.

It follows from the above that by means of the system the test can be carried out using at least one test object having a predetermined size. Thus according to the invention it is not required to use a plurality of test objects having mutually different sizes. In accordance with the invention it is even possible to use a plurality of test objects having one and the same size. Thus, in use, a magnification of the camera is selected which is just sufficient for being able to clearly view and/or recognize the at least one test object by means of the camera. Based on this selected magnification and other known parameters such as the size of the test object and a distance between the eye of the person and the test object when the test object is seen by the person by means of the portable camera, the recommended magnification factor and/or the recommended range for magnification factors can be calculated by means of the camera based on a predetermined algorithm. In this way the person can immediately learn the value of the calculated recommended magnification factor and use this to select his loupe of electronic magnifier.. The recommended magnification factor and/or the recommended range for magnification factors is preferably projected on the display of the camera. The camera may be provided with processing means for making the required calculations.

The size of the object may be for example the height of the object such as the height of the symbol. In that case the width of the object as seen by a person is about the same as its height so that its height and thereby its size indeed determines the visibility of the object. The symbol may for example be a character or a sign. Preferably it holds that the test object comprises at least one symbol having the predetermined size printed on a test sheet.

Preferably it holds that a plurality of test objects are provided, each comprising a symbol having the predetermined size wherein each of the symbols are printed on one test sheet. If a plurality of the test objects having one and the same predetermined size are used, it turns out to be more easy for a person to judge whether or not he can clearly view and/or recognize the test objects by means of the portable camera.
According to a preferred embodiment it holds that the camera is arranged to be positioned on the test sheet to be recorded wherein the camera is provided with an outer lower surface which is arranged to be in contact with the test sheet if the camera is positioned on the test sheet, and wherein if the outer lower surface is in contact with the test sheet there is a predetermined distance between the test sheet and the light sensitive sensor. The advantage of this embodiment is that the distance between the test sheet and the light sensitive sensor is fixed. Thereby the only distance which has to be observed is the distance between the eye and the camera. This makes that the system for calculating the recommended magnification factor and/or the recommended range for magnification factors can function in a very accurate way. Preferably it holds that the parameters comprise as an input parameter for calculating the recommended magnification factor and/or the recommended range for magnification factors: the selected magnification for the displayed at least one portion of the image, possibly the size of the at least one test object if this size may be varied and selected and possibly a distance between an eye of the person and the camera such as a distance between the eye and one of the optical sensor and the display while the at least one test object is seen by the person by means of the portable camera and if this distance may be varied and selected. If the size of the test object and/or the distance between an eye and the camera can not be selected and are fixed (because for example these parameters and prescribed in a manual of the system) these parameters can be fixed in the algorithm and are no input parameters but fixed parameters of the algorithm.

In accordance with a preferred embodiment it further holds that the parameters may also comprise as an input parameter for calculating the recommended magnification factor and/or the recommended range for magnification factors: the predetermined distance between the test sheet and the light sensitive sensor if this distance may be selected in advance and is not fixed, for example fixed by means of the camera. In accordance with a very special embodiment it holds that the portable camera is provided with at least one lighting unit for lighting the object to be recorded. More particularly the portable camera is provided with at least one lighting unit for lighting the test sheet to be recorded when the camera is positioned on the test sheet. In such a way the test can be performed very reliably because the test sheet is lighted in a predetermined manner. This means that the test according to which a person has to judge whether or not he can clearly view and/or recognize the test object by means of the portable camera can be carried out in a way wherein, if the test is repeatedly carried out, the same result is obtained. This is caused by the fact that the test sheet is lighted in a predetermined way by means of the lighting unit and the influence of (day) light in the environment of the test sheet is decreased..

Preferably it holds that the camera is provided with a first body portion, a second body portion and optionally a means for connecting and disconnecting the first body portion and the second body portion, wherein the second body portion is provided with the sensor and the display and wherein the first body portion is arranged to be positioned on an at least one at least one test object to be recorded while the second body portion is connected with the first body portion such that, in use, the first body portion provides a fixed distance between the second body portion and the object to be recorded wherein the first body portion is provided with a side wall surrounding an inner space of the first body portion and a first and second opening lying opposite to each other and each providing visible access to the inner space (if the first body portion and the second body portion are disconnected from each other) wherein if the first body portion and the second body portion are connected together the second opening faces the second body portion and the first opening, in use, faces the object to be recorded such that the sensor can capture an image of the at least one at least one test object to be recorded via the second opening, the space and the first opening. If the first and second body portions are connected, the camera can be used in a very reliable way for carrying out the test. Preferably it holds that the first body portion comprises the outer lower surface which is arranged to be in contact with the test sheet. Also preferably it holds that the second body portion is provided with a lighting unit. Because the first body portion not only provides a fixed distance between the second body portion and the object to be recorded, but also provides a shell against light from the environment of the test sheet the advantage is created that the lighting of the test sheet is only determined by the lighting unit and not by the light of the environment. Thereby the test becomes very reliable.

It is however also possible that the first body portion is removed from a second body portion wherein the second body portion is used for carrying out the test without that the camera is in contact with the test object such as a test sheet.

It is however also possible that the first body portion and the second body portion are attached to each other without the possibility that the first body portion and the second body portion can be separated from each other. The first body portion and the second body portion may in fact form an integrated unit wherein the housings of the first body portion and the second body portion form one and the same housing.

Further advantage aspects of the invention will become clear from the appended description of preferred embodiments. The invention will now be described in reference to the accompanying drawings, in which:
Fig. 1 is a schematic side view of a system according to the invention;
Fig. 2a shows a side view of a possible embodiment of a camera of a system according to the invention;
Fig. 2b shows a perspective view of the camera of fig. 2a;
Fig. 2c shows a second body portion of the camera of fig. 2a;
Fig. 3 shows a cross section of the camera of fig. 2a;
Fig. 4 shows a perspective view of the camera of fig. 2a in use;
Fig. 5 shows a perspective view of the second body portion of the camera of fig. 2a in accordance with an other use then in fig. 4; and
Fig. 6 shows a possible embodiment of a test sheet provided with symbols having a predetermined size.

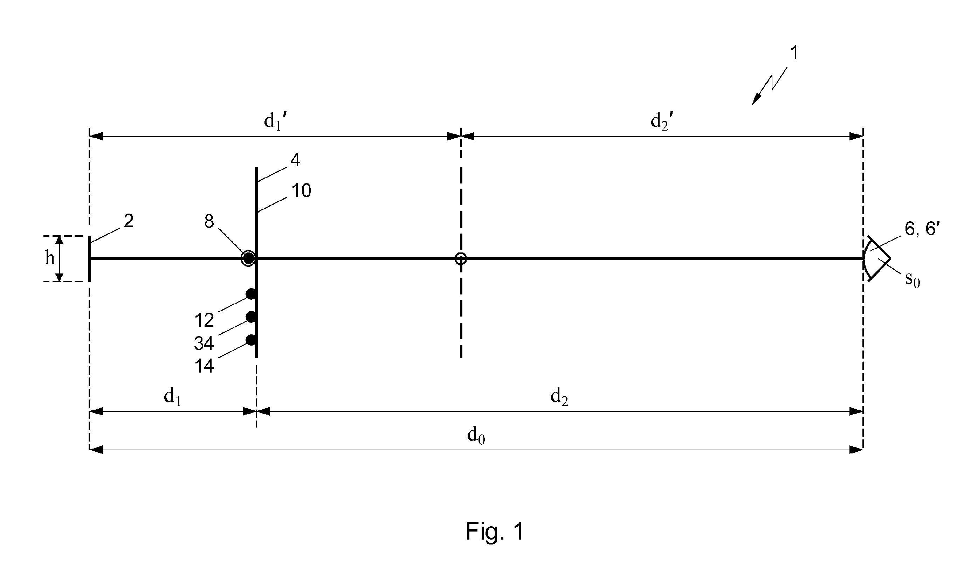

In fig. 1 a system for determining a recommended magnification factor for a magnifier such as a loupe or an electronic magnifier is indicated with reference number 1. The system comprises a test object 2 having a predetermined size h. The system further comprises a portable camera 4 for recording an image of at least a portion of the test object 2 and for displaying at least a portion of the image of the test object to a person. In fig. 1 the person's eye carries reference number 6. In this example the size of the object is the height of the object. In this case the width of the object is not greater than its height so that the height and thereby the size of the object determines the visibility of the object. The width may for example be about the same or smaller than its height.

The camera is provided with a light sensitive sensor 8 for obtaining an image of at least a portion of the test object 2. Furthermore, the camera is provided with a display 10 and processing means 12 for processing the image obtained by means of the sensor 8 and for displaying at least a portion of the image of the test object on the display 10. The test object may be displayed on the display in a possible magnified form. The camera is provided with means 14 for selecting the magnification of the at least one portion of the image which is displayed on the display. The means 14 may, for example, be a button for selecting the magnification. The means 14 may also be formed by the display 10 if the display 10 is formed by a touch screen. In the example of fig. 1 the distance between the eye 6 of the person and the test object 2 is d0. In this example, the distance between the camera 4 and the test object is d1 and the distance between the camera 4 and the eye 6 is d2. It may be appreciated that the person using one of his eyes 6 or both eyes 6, 6' can not clearly view and/or recognize the test object 2. If the person is however viewing the test object 2 via the portable camera on the display 10, the user may select a magnification of at least one portion of the image which is displayed on the display 10 such that the person can clearly view and/or recognize the test object by means of the portable camera 4. More particularly, he may select a magnification factor which is the lowest magnification whereby the person can clearly view and/or recognize the test object by means of the portable camera. It may be appreciated that this magnification together with the size of the test object 2 and the distance d0 between the eye and the test object 2 represents a collection of values which inherently represent a recommended magnification factor and/ or a range for magnification factors for a magnifier such as a loupe or an electronic magnifier to be used by a person for viewing objects having variable sizes as clearly as persons having good eyes which view the same objects. In other words, the recommended magnification factor and/ or the range for magnification factors is intended to be used by the person having a disabled view so that the person may clearly view the objects which can also be clearly viewed by persons having good eyes. According to the present invention, the camera is arranged to calculate the recommended magnification factor and/ or the range for magnification factors on the basis of a predetermined algorithm wherein the predetermined algorithm uses as input parameters: the selected magnification of the displayed at least one portion of the image, the size of the test object, and a distance d0 between an eye of the person and the test object while the test object is seen by the person by means of the camera and/or parameters which can be derived from the selected magnification for the displayed at least one portion of the image, the size of the test object and the distance between an eye of the person and the object while the object is seen by the person by means of the portable camera.

In accordance with a first approximation, the predetermined algorithm is independent from the distance d1 between the test object 2 and the camera 4 and the distance d2 between the camera 4 and the eye 6. In this first approximation the predetermined algorithm only depends on the distance d0=d1+d2. This can be understood as follows.

If the distance between the camera and the test object is d1, and the distance between the camera and the eye 6 is d2 and a certain magnification of the at least one portion of the image is selected, the test object will be projected within the eye with a certain size. This size is indicated by s0. If the distance d1 is increased up until d1' the size of the image of the test object on the display 10 will decrease. This will have a decreasing effect on the size of the image of the test object projected in the eye 6. However, at the same time the distance between the camera and the eye will decrease, which will have an increasing effect on the image of the test object in the eye. Thus, said decreasing and increasing effect on the image of the test object projected in the eye to a large extend compensate each other. This means that in a first approximation, the test for determining the recommended magnification factor can be carried out without exactly knowing the distances d1 and d 2 wherein it is however only required that the distance d0 is known. In that case the selected magnification m for the displayed at least one portion of the image, can be expressed in the form of a zoom factor of the camera.. Thus in this approximation the zoom factor, d0 and the actual size of the test object determine the size s0 of the projection of the test object in the eye 6. If the selected zoom factor is the smallest factor wherein a person can clearly view or recognise the test object this means that s0 is the smallest size of the object projected in the eye which can be clearly viewed or recognised, This size s0 represents a value for a recommended magnification factor for a loupe or an electronic magnifier to be used for, for example reading a magazine or book. This is based on the fact that it is generally known what the average size s1 of characters used in books and magazines is as well as the average reading distance d3 which provides a dioptric power 100/d3. Thus s1*100/d3 is a measure for the size of the average sized character as projected in the eye. Please note that this measure can be regarded as known and fixed. Therefore s0/(s1*100/d3) may provide a measure for the recommended magnification factor for a loupe or an electronic magnifier. Because s1 and d3 are known and fixed the recommended magnification factor only depends on s0. The predetermined algorithm which is used in this example is based on this view.

The predetermined algorithm may comprise in that case the following formula (A): RM=f(d0, m, h)= C1*(m*h)/d0, wherein d0 is the distance between an eye and the at least one test object, m is a value representing the selected magnification factor more particularly a selected zoom-factor of the camera, h is the size of the at least one test object, C1 is a constant which depends on how a (recommended) magnification of a camera is defined (for example zoom-factor, magnification factor etc.) and RM is the recommended magnification factor wherein m is an input parameter and d0 may be an input parameter for the predetermined algorithm. The formula is based on the following insights: If m is relatively large, the eye apparently needs relatively more magnification for proper viewing. If h is relatively large the eye apparently needs also relatively more magnification for proper viewing. If d0 is relatively large than the eyes apparently needs relatively less magnification for proper viewing. Thus RM increases if m and/or h increase and RM decreases if d0 increases. This is reflected by the formula (A) If the distance d0 is prescribed, for example a manual of the system, than d0 need not be an input parameter for the algorithm because d0 is fixed. In that case it may even be that d0 can not be changed and can not be entered into the camera by a user. Of course d0 will be hidden within the predetermined algorithm. If the distance d0 may be selected by the user it may be inputted in the camera by means of some input device of the camera such as buttons 14 and/or a touch screen. Of course m is an input parameter for the algorithm which can be supplied by the camera itself into the algorithm. Thus m need not be a parameter which is entered into the camera by the user. The user may simply select a smallest magnification factor whereby he van clearly view and/or recognise the test object by means of the camera without knowing what the value of m actually is. The actual value is may only be known within the camera and be used by the camera for making calculations in accordance with the predetermined algorithm. Once the camera has calculated the value of RM is may be displayed on the display 10. It may also be that the camera is provided with a plurality of ranges for recommended magnification such as range A= 1-3, range B=3-6 and range C=6-9. It may be that the camera determines in which range RM falls and than displays information about the range on the display in addition to or in stead of the calculated RM. Thus in that case the predetermined algorithm provided and recommended range for magnification factors which range is shown on the display 10. It is noted that the camera may be provided with processing means 34 for carrying out the predetermined algorithm.

The system can be used in a more accurate way if the distance d1 is predetermined and fixed. In that case, a magnification factor of the camera can be selected which directly indicates the ratio between the size of the object 2 as shown on the display 10 and the real-size of the object 2. Thus, in that case the selected magnification of the at least one portion of the image which is displayed on the display is not a zoom factor of the camera but the ratio between the size h' of the object as shown on the display and the real size h of the object 2. This ratio is also referred to as an enlargement factor. It will be appreciated that the size of the image of the test object as projected in the eye, not only depends on the selected magnification factor of the camera, which is in this example the enlargement factor, but also on the distance d2. This distance d2 provides a dioptric power which equals to 100/d2 wherein d2 is measured in centimeters. Thus the enlargement factor, d2 and the actual size of the test object determine the size s0 of the projection of the test object in the eye 6. Thus in this approximation the enlargement factor m, d2 and the actual size of the test object determine the size s0 of the projection of the test object in the eye 6. If the selected enlargement factor m is the smallest factor wherein a person can clearly view or recognise the test object this means that s0 is the smallest size of the object projected in the eye which can be clearly viewed or recognised, This size s0 represents a value for a recommended magnification factor for a loupe or an electronic magnifier to be used for, for example reading a magazine or book. This is based on the fact that it is generally known what the average size s1 of characters used in books and magazines is as well as the average reading distance d3 which provides a dioptric power 100/d3. Thus s1*100/d3 is a measure for the size of the average sized character as projected in the eye. Please note that this measure can be regarded as known and fixed. Therefore s0/(s1*100/d3) may provide a measure for the recommended magnification factor for a loupe or an electronic magnifier. Because s1 and d3 are known and fixed the recommended magnification factor only depends on s0. The predetermined algorithm which is used in this example, wherein d1 is known and fixed, is also based on this view.
It holds that s0=C2*(100/d2)*m*(100/d1)*h wherein C2 is a constant depending on the characteristics of the human eye (and on whether or not the eye is equipped with lenses or glasses) and (100/d2) and (100/d1) are dioptric powers and wherein m is a factor which indicates a zoom-factor of the camera. Thus it holds that RM=C3*C2*(100/d2)*m*(100/d1)*h=C4*(100/d2).m.(100/d1)*h wherein C3 is a constant depending on how the magnification of camera is classified (depending on which units are used for classifying the camera such as zoom factor or a magnification factor) and depending on s1 and d3 wherein C4=.C3*C2. It is noted that RM=C4*(100/d1)*m*(100/d2)*h is a formula (B) based on which other formulas can be derived. For example because d0=d1+d2 an approximation of this formula (B) is RM=C1*m*(1/(d1+d2)*h, the formula (A) used above.

The predetermined algorithm which is used in this example, wherein d1 is known and fixed, is also based on this formula (B). In that case, the predetermined algorithm comprises the formula RM=f(d2, m, h)= C4*(100/d2)*m*(100/d1)*h=C5*(100/d2)*m*h wherein C5=C4*(100/d1), m represents the zoom-factor which is in this case also a measure of the magnification factor because d1 is known and fixed. wherein d2 is the distance between the camera and the eye. Thus m is the selected magnification factor, more particularly the ratio between the size of the object and the size of the object as displayed on the display (also referred to as enlargement factor)_, h is the size of the at least one test object and RM is the recommended magnification factor and wherein m is an input parameter. Similarly as discussed above d2 may be an input parameter for the algorithm or fixed and h may be an input parameter for the algorithm or fixed. If d2 would also be known and fixed it follows that
RM=C4*(100/d1)*m*(100/d2)*h=C6*m, wherein C6= C4*(100/d1)*(100/d2)*h. Thus in that case RM only depends on m because C6 is known and fixed and it holds that RM=f(m).
In yet another embodiment RM=C4*100/D2*M wherein M=(hs/h)/(dr/d1) wherein dr is a reference distance between the camera and the object and wherein hs is the size, in this example the height of the object on the display.

According to another possibility the distance d1 between the camera and the test object is not fixed but known. Then the selective magnification factor can again be the zoom factor of the camera. In combination with d1 this zoom factor provides information about the ration between the size of the at least one test object as displayed on the display and the actual the size of the test object.. Furthermore 100/d2 provides again information about the dioptric power of the working distance d2. Again this dioptric power equals 100/d2 if d2 is measured in centimeters. Thus, indeed, based on the distance d1, the zoom factor m of the camera, the size of the test object and d2 the size s0 can be determined. If the zoom factor is the smallest factor wherein a person can clearly view or recognise the test object this means that s0 is the smallest size of the object projected in the eye which can be clearly viewed or recognised, As explained above this size s0 represents a value of a recommended magnification factor for a loupe or an electronic magnifier to be used for, for example reading a magazine or book. The predetermined algorithm is based on this view. Thus in this case the zoom factor m, d1, d2 and the actual size of the test object determine the size s0 of the projection of the test object in the eye 6. If the selected zoom factor is the smallest factor wherein a person can clearly view or recognise the test object this means that s0 is the smallest size of the object projected in the eye which can be clearly viewed or recognised, This size s0 represents a value for a recommended magnification factor for a loupe or an electronic magnifier to be used for, for example reading a magazine or book. This is based on the fact that it is generally known what the average size s1 of characters used in books and magazines is as well as the average reading distance d3 which provides a dioptric power 100/d3. Thus s1*100/d3 is a measure for the size of the average sized character as projected in the eye. Please note that this measure can be regarded as known and fixed. Therefore s0/(s1*100/d3) may provide a measure for the recommended magnification factor for a loupe or an electronic magnifier. Because s1 and d3 are known and fixed the recommended magnification factor only depends on s0. The predetermined algorithm which is used in this example is based on this view. For example, it may hold that RM = f(d1, d2, m, h)= C4*(100/d2)*m*(100/d1)*h wherein m the selected magnification factor of the camera, in this example the zoom factor.

In accordance with the invention, the above referred to calculations are carried out by the camera. Thus, the camera is arranged to calculate the recommended magnification factor on the basis of the predetermined algorithm. For this, the processing means for processing the image may also be used for calculating the recommended magnification factor. Also the camera may be provided with separate processing means 34 for making this calculations. The calculated recommended magnification factor is in this example also displayed on the display 10. It is noted that in each of the above examples wherein the camera is schematically shown the distance d1 may also be referred to as the distance between the test object and the light sensitive sensor and the distance d2 may also be referred to as the distance between the display 10 and the eye 6.

In fig. 1 the test object may, for example, be a black upstanding bar having a width which is smaller then its' height h. The height h may for example, be 2 cm. if the distance d1 is about 2 m. If the distance d1 is however for example, in the range of 5-10 cm. the height h may be for example be 1-2 mm. Please note that these are merely examples; other values for d1 and h are also possible. The value of d2 may for example be in the range of 20-75 cm. The height of the object represents the size of the object.

Preferably the test object comprises at least one symbol having the predetermined size h printed on a test sheet. Please note that a symbol may also be a character, but a symbol is not limited to characters. Therefore a symbol may also be a sign, a drawing of a product, men or animal, which drawing has for example as a height the predetermined size h. Preferably however, the at least one symbol is a character. Even more preferably a plurality of test objects are provided wherein each test object comprises a symbol having the predetermined size and wherein each of the symbols are printed on one test sheet. In other words, a single test sheet may be used wherein a plurality of symbols are shown, having the same size. This has as an advantage that the person who is doing the test does not have to concentrate on a single symbol but can more easily decide which magnification of the symbols has to be selected so that each of these symbols can be cleared viewed and/or recognized.

In view of the above it is clear that the method according to the invention comprises the step of selecting a distance between the eye of the user and the at least one test object for viewing the at least one test object by the person by means of the camera and/or the step of selecting a distance between the camera and an eye of the person and possibly selecting a distance between the at least one test product and the camera if this distance is not predetermined by the camera and wherein the method further comprises selecting a lowest magnification factor whereby the person can clearly view the at least one test object and/or can recognise the at least one test object and calculating the recommended magnification factor and/or the recommended range for magnification factors using the predetermined algorithm on the basis of the size of the at least one test object, a value representing the selected lowest magnification factor and the distance between the eye and the at least one test object or the distances between the eye and the camera and the at least one test object and the camera.

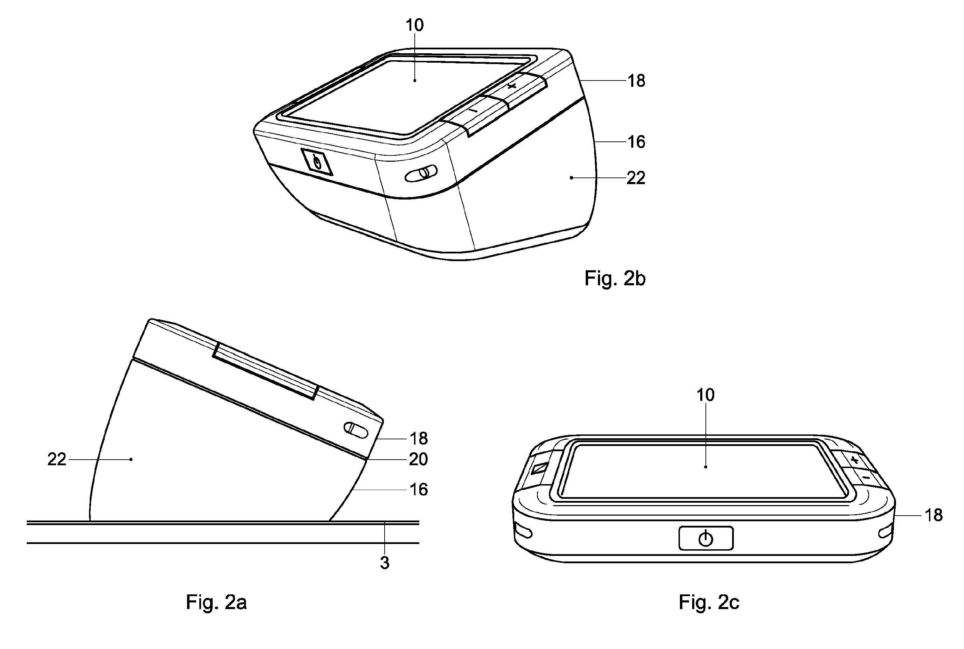

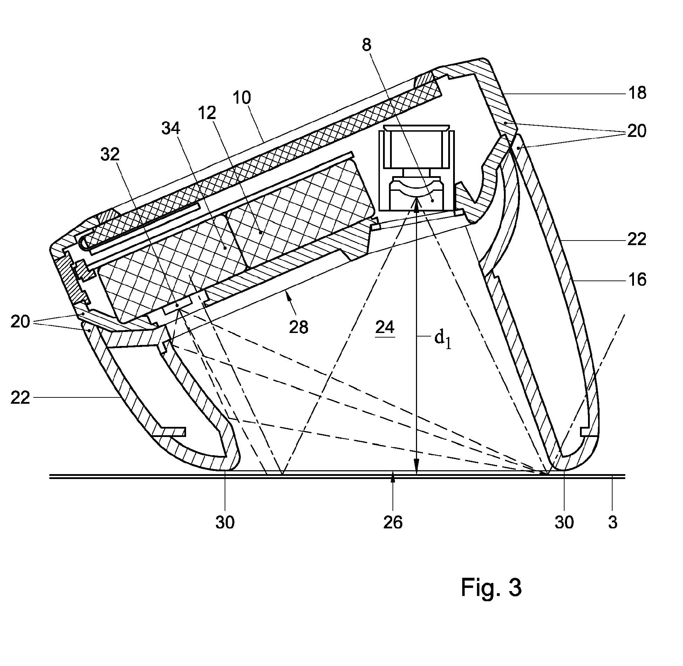

In fig. 1 the camera was only shown in a schematic manner. Fig. 2a-5 show a practical embodiment of the camera. The portable camera 4 is provided with a first body portion 16, a second body portion 18 and optionally a means 20 for connecting and disconnecting the first body portion and the second body portion. In this example the camera is provided with this connection and disconnection means 20 in the form of a magnet which is provided in the first body portion and a metal ball which is provided in the second portion. The magnet and metal ball are indicated in figure 3.

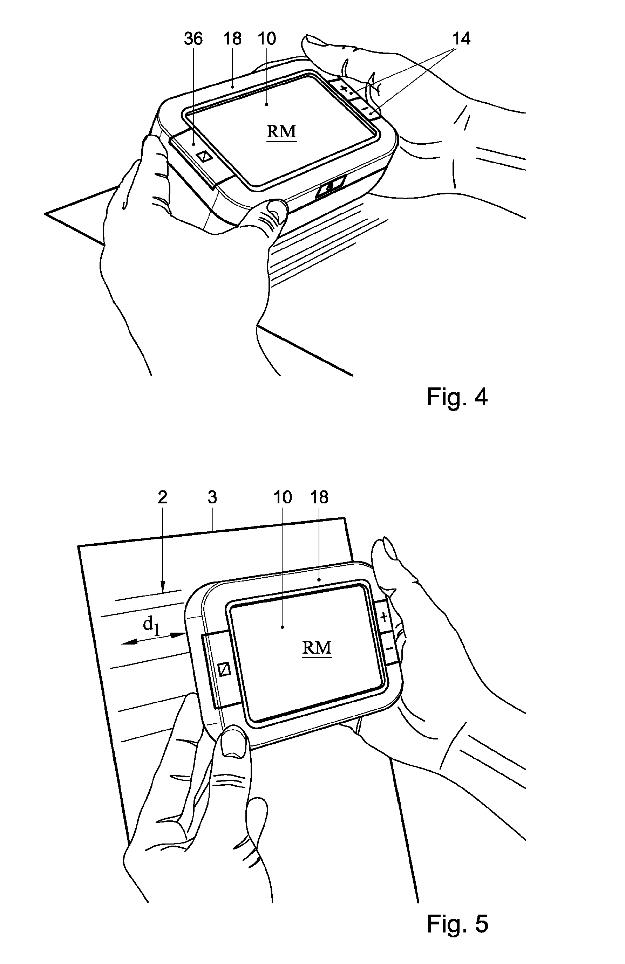

The second body portion is provided with a light sensitive sensor 8 and the display 10. The first body portion 16 is arranged to be positioned on the at least one test object 2 to be recorded while the second body portion is connected with the first body portion. Thus, in use, the first body portion 16 provides a fixed distance d1 (see fig. 3) between the sensor of the second body portion and the object to be recorded. The first body portion 16 is provided with a side wall 22 surrounding an inner space 24 of the first body portion. The first body portion is further provided with a first and second opening 26, 28 laying opposite to each other wherein each opening provides visible access to the inner space 24 if the first body portion and the second body portion are disconnect from each other. If the first body portion and the second body portion are connected together, the second opening 28, in use, faces, and is adjacent to, the second body portion and the first opening, in use, faces and is adjacent to the object to be recorded. As a result, the sensor 8 can capture an image of the at least one test object to be recorded via the second opening 28, the space 24 and the first opening 26. In this example a plurality of test objects are provided each comprising a symbol having the predetermined size h wherein each of the symbols are printed on one sheet 3 which is referred to as the test sheet 3. The first body portion comprises an outer surface 30. The camera is arranged to be positioned on the test sheet 3 to be recorded, wherein the camera is provided with the outer surface 30. The outer surface is arranged to be in contact with the test sheet. If the outer surface is in contact with the test sheet 3, there is a predetermined distance d1 between the test sheet 3 and the light sensitive sensor 8 and thereby between the at least one test object 2 and the sensor 8. The second body portion 18 is further provided with a lighting unit 32 for lighting, in use, the test sheet 3. Because, in use, the test sheet 3 is only lighted by means of the lighting unit 32, and is not lighted by means of daylight (due to the fact that the space 24 is surrounded by the side walls 22 and the second body portion), the lighting of the test sheet is always in a predetermined known manner which makes testing very reliable. The processing means 12 for processing the image obtained by means of the sensor and for displaying at least a portion of the image on the display 10, is also present in the second body portion. The second body portion also comprises processing means 34 for calculating the recommended magnification factor and thus for carrying out the predetermined algorithm. In use, the camera is positioned on a test sheet 3 to be recorded as shown in fig. 4. The camera is provided with buttons 34 for varying the magnification of the at least one portion of the image which is displayed on a display 10. Because the distance d1 is predetermined and fixed the selected magnification can be expressed in the ratio (enlargement factor) between the size of the symbols as displayed on the display 10 and the actual size of the symbols. In use, the user selects by means of the buttons 34 the smallest required ratio to clearly view and/or recognize the symbols. Based on this ratio, the recommended magnification RM can be calculated on the basis of the formula RM=f(d2, m, h) = C5*(100/d2)*m*h. In this formula m is an input parameter which is automatically used in the algorithm by means of the camera. If the size of the test object and thus in this case the size of the symbols is predetermined and always the same, h may be fixed in the formula and therefore is not an input parameter. Similarly if the distance d2 between an eye of the person from which the eye is tested and the display is fixed because for example a manual of the camera requires this distance to be fixed, then also d2 is fixed and is not an input parameter for the predetermined algorithm. Moreover, in case d2 and h may be varied, a user has to be able to input the value of d2 and h in the camera. This can, for example, be carried out by using the buttons 14 in combination with a menu button 36. By pushing the menu button 36 a section for inputting d2 and h may be selected. It will be clear that if d2 and h are fixed those parameters will be present in the predetermined algorithm but may not be recognized as such within the predetermined algorithm as a fixed parameter. In this example the calculated recommended magnification factor RM is displayed on the display 10. The display 10 also shows in its lower part four lines of characters wherein each character is a symbol having the same predetermined height h and each symbol forms a test object.

The second body portion 18 may also be separated from the first body portion. By means of the second body portion 18, a similar test may be carried out. In that case, the test sheet 3 comprising the plurality of test objects may for example be stuck to a wall of a chamber. Based on the manual of the camera, it may be that the distance d1 is again predetermined. This means that a user must hold the camera as shown in fig. 5 at a predetermined distance from the test sheet 3. In that case, again, the predetermined algorithm may comprise the formula RM= f(d2, m, h)= C5*(100/d2)*m*h wherein m is, again, an input parameter which indicates the ratio between the size of the test object as shown on the display 10 and the actual size of the test object as printed on the test sheet 3. Again d2 and h may be fixed if this is required by the manual of the camera. Thus, in that case, m is the only input parameter. It is however also possible that a user may vary and select its' own test sheet having a different size of symbols so that h is an input parameter. Also the distance d2 between an eye of the person and the display 10 may be pre-described in the manual so that d2 is not an input parameter. It is however also possible that d2 forms an input parameter if the d2 can be selected by the user.

If however, also d1 is an input parameter which may be selected by the user, the predetermined algorithm may comprise the following formula: RM = f(d1, d2, m, h)= C4*(100/d2)*m*(100/d1)*h. In that case, first the input parameters d1, d2 and h have to be inputted by the user. The selected magnification factor m is in this case a zoom factor and is inputted after being selected into the algorithm by means of the camera itself. The factor m is a zoom factor and not an enlargement factor because the ratio between the size of the test object as shown on the display and the actual size of the test object depends on d1 and can therefore not be an input parameter. Based on the latest referred to formula, the value of the recommended magnification factor can be calculated and displayed on the display a shown in fig. 5.

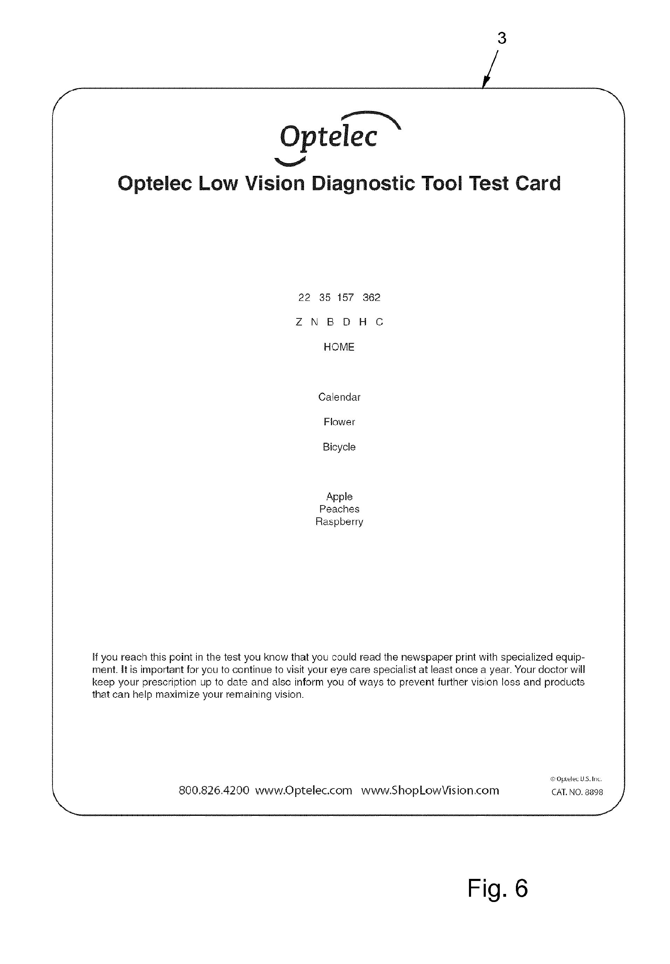

Figure 6 shows a possible embodiment of a test sheet 3. Other symbols and characters with other sizes are also possible. In each of the embodiments it holds that the recommended magnification factor can be used for selecting a suitable loupe or electronic magnifier to be used by a person for being able to comfortable reading. Such loupes and electronic magnifiers are known as such and are usually kept at a predetermined distance from the object such as a paper or a magazine which has to be read. More particularly the recommended magnification factor is the ratio between the size of the original symbols which must be read and the size of these symbols as displayed on the display of the electronic magnifier. In case of a loupe, it may just be the dioptre of the loupe.

In this example the processing means 12 is used for selecting the magnification factor m (digital zoom). It may however also be that the light sensitive sensor 8 provides an adjustable magnification because it is a zoom lens. In this example the test is carried out for only one eye at the time. It is also possible that the test is carried out while using both eyes. In the example RM is displayed on the display. It is also possible that the calculated. range of recommended magnification factors is displayed on the display. In this example formulas are used for carrying out the calculations. In accordance with the invention a calculation is understood to cover also selecting the value of RM from a table based on the relevant input parameters such as m, h, d1 and d2. If some of these parameters are fixed and known the selection in the table may be based only on the parameters which vary such as m. The table may be stored in the camera, more specifically in the processing means 12 or 34 of the camera. Also in this example RM is determined on the basis of one eye. Of course RM can also be determined in the same way as explained above while using both eyes. In the above examples the size of the object is the height of the object. This both holds for the actual size of the object as well as for the size of the object on the display of the camera. In this case the width of the object is for example not greater than its height so that the height of the object determined the visibility of the object. The width may for example be about the same as its height or smaller. It is however possible to express the size of the object in other ways. If the object would be a circle it good be its diameter, Such varieties all fall within the scope of the present invention.

The claim set as filed in the original Dutch application is repeated now as clauses in order to include all original subject matter in the present application: 18. A method for determining a recommended magnification factor and/or the recommended range for magnification factors for a magnifier such as a loupe or an electronic magnifier to be used by a person wherein the method comprises the use of at least one test object having a predetermined size and a portable camera for recording an image of at least a portion of the at least one test object and displaying at least a portion of the image of the at least one test object to the person, wherein the camera is provided with a light sensitive sensor for obtaining an image of at least a portion of the at least one test object, a display and processing means for processing the image obtained by means of the sensor and for displaying at least a portion of the image of the at least one test object on the display, in possible a magnified form, wherein the camera is provided with means for selecting the magnification of the at least one portion of the image which is displayed on the display and wherein the camera is arranged to calculate the recommended magnification factor and/or the recommended range for magnification factors on basis of a predetermined algorithm wherein the predetermined algorithm uses as input parameters: a value representing the selected magnification for the displayed at least one portion of the image, the size of the at least one test object and a distance between an eye of the person and the at least one test object while the at least one test object is seen by the person by means of the portable camera; and/or the recommended range for magnification factors is value representing the selected magnification for the displayed at least one portion of the image, the size of the at least one test object and a distance between an eye of the person and the at least one test object while the object is seen by the person by means of the portable camera wherein the method comprises the step of selecting a distance between the eye of the user and the at least one test object for viewing the at least one test object by the person by means of the camera and/or the step of selecting a distance between the camera and an eye of the person and possibly selecting a distance between the at least one test product and the camera if this distance is not predetermined by the camera and wherein the method further comprises selecting a lowest magnification factor whereby the person can clearly view the at least one test object and/or can recognise the at least one test object and calculating the recommended magnification factor and/or the recommended range for magnification factors using the predetermined algorithm on the basis of the size of the at least one test object, a value representing the selected lowest magnification factor and the distance between the eye and the at least one test object or the distances between the eye and the camera and the at least one test object and the camera.
19. Method according to clause 18, characterized in that, the value representing the selected magnification for the displayed at least one portion of the image is automatically used by the camera in the predetermined algorithm without being inputted by a user.
20. Method according to clause 18 or 19, characterised in that the at least one test object is at least one symbol having the predetermined size wherein the at least one symbol is printed on a test sheet.
21. Method according to clause 20, characterised in that a plurality of test objects are used each comprising a symbol having the predetermined size wherein each of the symbols are printed on one test sheet.
22. Method according to clause 20 or 21, characterised in that the camera is positioned on the test sheet to be recorded wherein the cameras is provided with an outer lower surface which is in contact with the test sheet and wherein if the outer lower surface is in contact with the test sheet there is a predetermined distance between the test sheet and the camera such as a predetermined distance between the test sheet and the light sensitive sensor.
23. Method according to clause 22, characterised in that the parameters comprise as an input parameter for calculating the recommended magnification factor and/or the recommended range for magnification factors: the selected magnification for the displayed at least one portion of the image, possibly the size of the at least one test object if this size may be varied and is selected in advance and possibly a distance between an eye of the person and the camera such as a distance between the eye and one of the optical sensor and the display while the at least one test object is seen by the person by means of the portable camera of this distance may be varies and is selected in advance.
24. Method according to clause 23, characterised in that the parameters may also comprise as an input parameter for calculating the recommended magnification factor and/or the recommended range for magnification factors: the predetermined distance between the test sheet and the camera such as a predetermined distance between the test sheet and the light sensitive sensor if this distance may be selected in advance.
25. Method according to any preceding clause 18-24, characterized in that the calculated recommended magnification factor is displayed on the display.
26. Method according to any preceding clause 18-25, characterized in that the method comprises selecting a lowest magnification factor whereby the person can clearly view the at least one test object with predefined first criteria and/or can recognise the at least one test object with predefined second criteria.
27. Method according to any preceding clause 18-26, characterized in that the at least one test object is lighted by means of at least one lighting unit of the camera.
28. Method according to clause 22, 23 or 24 and according to clause 27, characterized in that that, the test sheet is lighted by means of the at least one lighting unit when the camera is positioned on the test sheet.
29. Method according to any preceding clause 18-28, characterised in that the magnification of the recorded image is varied by means of the processing means and/or an optical zoom of the light sensitive sensor for selecting the lowest magnification.
30. Method according to any preceding clause 18-29, characterized in that the predetermined algorithm is carried out by means of processing means of the camera.
31. Method according to any preceding clause 18-30, characterised in that the camera is provided with a first body portion, a second body portion and optionally a means for connecting and disconnecting the first body portion and the second body portion, wherein the second body portion is provided with the sensor and the display wherein the first body portion is provided with a side wall surrounding an inner space of the first body portion and a first and second opening lying opposite to each other and each providing visible access to the inner space (if the first body portion and the second body portion are disconnected from each other) wherein if the first body portion and the second body portion are connected together the second opening faces the second body portion and the first opening, in use, faces the at least one test object to be recorded such that the sensor can capture an image of the at least one test object to be recorded via the second opening, the space and the first opening.
32. Method according to clause 31, characterised in that the first body portion is positioned on the test sheet to be recorded while the second body portion is connected with the first body portion such that, in use, the first body portion provides a fixed distance between the second body portion and the test sheet to be recorded
33. Method according to clauses 22 and 32, characterized in that first body portion comprises the outer lower surface.
34. Method according to clause 27 or 28 and clause 31, 32 or 33, characterised in that the second body portion is provided with the lighting unit.
35. Method according to any preceding clause 18-34, characterised in that the predetermined algorithm comprises the following formula: RM=f(do, m, h)= C1*(m*h)/d0, wherein d0 is the distance between an eye and the at least one test object, m is a value representing the selected magnification factor more particularly a zoom-factor of the camera, h is the size of the at least one test object, C1 is a constant and RM is the recommended magnification factor or an magnification factor on the basis of which the recommended range for magnification factors is selected and wherein m is an input parameter, d0 may be an input parameter or fixed and h may be an input parameter or fixed; or if the distance d1 between the camera and the at least one test object is fixed RM=f(d2, m, h)= C5*(100/d2)*m*h, wherein d2 is the distance between the camera and the eye which may also be expressed as the distance between the display and the eye, m is the selected magnification factor more particularly the ratio between the size of the object and the size of the object as displayed on the display, h is the size of the at least one test object, C5 is a constant and RM is the recommended magnification factor or an magnification factor on the basis of which the recommended range for magnification factors is selected and wherein m is an input parameter, d2 may be an input parameter or fixed and h may be an input parameter or fixed; or
   RM=f(d1, d2, m, h)= C4*(100/d2)*m*(100/d1)*h, wherein d1 is the distance between the at least one test object and the camera which may also be expressed as the distance between the test object and the light sensitive sensor, d2 is the distance between the camera and the eye which may also be expressed as the distance between the display and the eye, m is the selected magnification factor more particularly the ratio between the size of the object and the size of the object as displayed on the display, h is the size of the at least one test object, C4 is a constant and RM is the recommended magnification factor or an magnification factor on the basis of which the recommended range for magnification factors is selected and wherein m is an input parameter, d1 may be an input parameter or fixed, d2 may be an input parameter or fixed and h may be an input parameter or fixed; or
   if the distance between the camera and eye is fixed, the distance between the object and the camera is fixed and the size of the object is fixed RM=f(m)=C6*m, wherein C6 is a constant and m is the selected magnification factor more particularly the ratio between the size of the object and the size of the object as displayed on the display; or RM=C4*100/d2*M wherein M=(hs/h)/(dr/d1) wherein dr is a reference distance between the camera and the object and wherein hs is the size of the at least one test object on the display, C4 is a constant, h is the size of the at least one test object, d1 is the distance between the object and the camera and d2 is the distance between the eye and the camera.

## Claims

1. A system for determining a recommended magnification factor and/or a recommended range for magnification factors for a magnifier such as a loupe or an electronic magnifier to be used by a person wherein the system comprises at least one test object having a predetermined size and a portable camera for recording an image of at least a portion of the at least one test object and displaying at least a portion of the image of the at least one test object to the person, wherein the camera is provided with a light sensitive sensor for obtaining an image of at least a portion of the at least one test object, a display and processing means for processing the image obtained by means of the sensor and for displaying at least a portion of the image of the at least one test object on the display, in possible a magnified form, wherein the camera is provided with means for selecting the magnification of the at least one portion of the image which is displayed on the display and wherein the camera is arranged to calculate the recommended magnification factor and/or the recommended range for magnification factors on basis of a predetermined algorithm wherein the predetermined algorithm uses as parameters: a value representing the selected magnification for the displayed at least one portion of the image, the size of the at least one test object and a distance between an eye of the person and the at least one test object while the at least one test object is seen by the person by means of the portable camera; and/or parameters which can be derived from the value representing the selected magnification for the displayed at least one portion of the image, the size of the at least one test object and a distance between an eye of the person and the object while the at least one test object is seen by the person by means of the portable camera.

2. System according to claim 1, **characterized in that** the value representing the selected magnification for the displayed at least one portion of the image is automatically used by the camera in the predetermined algorithm without being inputted by a user.

3. System according to claim 1 or 2, **characterised in that** the at least one test object comprises at least one symbol having the predetermined size printed on a test sheet.

4. System according to claim 3, **characterised in that** a plurality of test objects are provided each comprising a symbol having the predetermined size wherein each of the symbols are printed on one test sheet.

5. System according to claim 3 or 4, **characterised in that** the camera is arranged to be positioned on the test sheet to be recorded wherein the camera is provided with an outer lower surface which is arranged to be in contact with the test sheet if the camera is positioned on the test sheet, and wherein if the outer lower surface is in contact with the test sheet there is a predetermined distance between the test sheet and the camera such as a predetermined distance between the test sheet and the light sensitive sensor.

6. System according to any preceding claim, **characterised in that** the parameters comprise as an input parameter for calculating the recommended magnification factor and/or the recommended range for magnification factors: the selected magnification for the displayed at least one portion of the image, possibly the size of the at least one test object if this size may be selected and possibly a distance between an eye of the person and the camera such as a distance between the eye and one of the optical sensor and the display while the at least one test object is seen by the person by means of the portable camera and if this distance may be selected.

7. System according to claim 6, **characterised in that** the parameters may also comprise as an input parameter for calculating the recommended magnification factor: the distance between the test object and the camera such as the distance between the test object and the light sensitive sensor if this distance may be selected in advance.

8. System according to any preceding claim, **characterized in that** the calculated recommended magnification factor and/or the recommended range for magnification factors is displayed on the display.

9. System according to any preceding claim, **characterized in that** the selected magnification is the lowest magnification whereby the person can clearly view and/or recognise the at least one test object by means of the portable camera.

10. System according to any preceding claim, **characterized in that** the portable camera is provided with at least one lighting unit for lighting the at least one test object to be recorded.

11. System according to claims 3, 4 or 5 and according to claim 9, **characterized in that** that, the portable camera is provided with at least one lighting unit for lighting the test sheet to be recorded when the camera is positioned on the test sheet.

12. System according to any preceding claim, **characterised in that** the magnification of the recorded image can be varied by means of the processing means and/or an optical zoom of the light sensitive sensor.

13. System according to any preceding claim, **characterized in that** the camera is provided with processing means for carrying out the predetermined algorithm.

14. System according to any preceding claim, **characterised in that** the camera is provided with a first body portion, a second body portion and optionally a means for connecting and disconnecting the first body portion and the second body portion, wherein the second body portion is provided with the sensor and the display and wherein the first body portion is arranged to be positioned on the at least one test object to be recorded while the second body portion is connected with the first body portion such that, in use, the first body portion provides a fixed distance between the sensor of the second body portion and the object to be recorded wherein the first body portion is provided with a side wall surrounding an inner space of the first body portion and a first and second opening lying opposite to each other and each providing visible access to the inner space (if the first body portion and the second body portion are disconnected from each other) wherein if the first body portion and the second body portion are connected together the second opening faces the second body portion and the first opening, in use, faces the object to be recorded such that the sensor can capture an image of the at least one test object to be recorded via the second opening, the space and the first opening.

15. A system according to claims 5 and 14, **characterized in that** first body portion comprises the outer lower surface.

16. System according to claim 10 or 11 and claim 14 or 15, **characterised in that** the second body portion is provided with the lighting unit.

17. System according to any proceeding claim, **characterised in that** the predetermined algorithm comprises the following formula:
RM=f(do, m, h)= C1*(m*h)/d0, wherein d0 is the distance between an eye and the at least one test object, m is a value representing the selected magnification factor more particularly a zoom-factor of the camera, h is the size of the at least one test object, C1 is a constant and RM is the recommended magnification factor or an magnification factor on the basis of which the recommended range for magnification factors is selected and wherein m is an input parameter, d0 may be an input parameter or fixed and h may be an input parameter or fixed; or
if the distance d1 between the camera and the at least one test object is fixed RM=f(d2, m, h)= C5*(100/d2)*m*h, wherein d2 is the distance between the camera and the eye which may also be expressed as the distance between the display and
the eye, m is the selected magnification factor more particularly the ratio between the size of the object and the size of the object as displayed on the display, h is the size of the at least one test object, C5 is a constant and RM is the recommended magnification factor or an magnification factor on the basis of which the recommended range for magnification factors is selected and wherein m is an input parameter, d2 may be an input parameter or fixed and h may be an input parameter or fixed; or
RM=f(d1, d2, m, h)= C4*(100/d2)*m*(100/d1)*h, wherein d1 is the distance between the at least one test object and the camera which may also be expressed as the distance between the test object and the light sensitive sensor, d2 is the distance between the camera and the eye which may also be expressed as the distance between the display and the eye, m is the selected magnification factor more particularly the ratio between the size of the object and the size of the object as displayed on the display, h is the size of the at least one test object, C4 is a constant and RM is the recommended magnification factor or an magnification factor on the basis of which the recommended range for magnification factors is selected and wherein m is an input parameter, d1 may be an input parameter or fixed, d2 may be an input parameter or fixed and h may be an input parameter or fixed; or
if the distance between the camera and eye is fixed, the distance between the object and the camera is fixed and the size of the object is fixed RM=f(m)=C6*m, wherein C6 is a constant and m is the selected magnification factor more particularly the ratio between the size of the object and the size of the object as displayed on the display; or RM=C4*100/D2*M wherein M=(hs/h)/(dr/d1) wherein dr is a reference distance between the camera and the object and wherein hs is the size of the at least one test object on the display, C4 is a constant, h is the size of the at least one test object, d1 is the distance between the object and the camera and d2 is the distance between the eye and the camera...

18. A method for determining a recommended magnification factor and/or the recommended range for magnification factors for a magnifier such as a loupe or an electronic magnifier to be used by a person wherein the method comprises the use of at least one test object having a predetermined size and a portable camera for recording an image of at least a portion of the at least one test object and displaying at least a portion of the image of the at least one test object to the person, wherein the camera is provided with a light sensitive sensor for obtaining an image of at least a portion of the at least one test object, a display and processing means for processing the image obtained by means of the sensor and for displaying at least a portion of the image of the at least one test object on the display, in possible a magnified form, wherein the camera is provided with means for selecting the magnification of the at least one portion of the image which is displayed on the display and wherein the camera is arranged to calculate the recommended magnification factor and/or the recommended range for magnification factors on basis of a predetermined algorithm wherein the predetermined algorithm uses as input parameters: a value representing the selected magnification for the displayed at least one portion of the image, the size of the at least one test object and a distance between an eye of the person and the at least one test object while the at least one test object is seen by the person by means of the portable camera; and/or the recommended range for magnification factors is value representing the selected magnification for the displayed at least one portion of the image, the size of the at least one test object and a distance between an eye of the person and the at least one test object while the object is seen by the person by means of the portable camera wherein the method comprises the step of selecting a distance between the eye of the user and the at least one test object for viewing the at least one test object by the person by means of the camera and/or the step of selecting a distance between the camera and an eye of the person and possibly selecting a distance between the at least one test product and the camera if this distance is not predetermined by the camera and wherein the method further comprises selecting a lowest magnification factor whereby the person can clearly view the at least one test object and/or can recognise the at least one test object and calculating the recommended magnification factor and/or the recommended range for magnification factors using the predetermined algorithm on the basis of the size of the at least one test object, a value representing the selected lowest magnification factor and the distance between the eye and the at least one test object or the distances between the eye and the camera and the at least one test object and the camera.

19. Method according to claim 18, **characterised in that** the camera is provided with a first body portion, a second body portion and optionally a means for connecting and disconnecting the first body portion and the second body portion, wherein the second body portion is provided with the sensor and the display wherein the first body portion is provided with a side wall surrounding an inner space of the first body portion and a first and second opening lying opposite to each other and each providing visible access to the inner space (if the first body portion and the second body portion are disconnected from each other) wherein if the first body portion and the second body portion are connected together the second opening faces the second body portion and the first opening, in use, faces the at least one test object to be recorded such that the sensor can capture an image of the at least one test object to be recorded via the second opening, the space and the first opening.

20. Method according to claim 19, **characterised in that** the first body portion is positioned on the test sheet to be recorded while the second body portion is connected with the first body portion such that, in use, the first body portion provides a fixed distance between the second body portion and the test sheet to be recorded.
